# EUROPÄISCHE PATENTSCHRIFT

(11) **EP 0 848 624 B1**
(45) Veröffentlichungstag und Bekanntmachung des Hinweises auf die Patenterteilung: **26.10.2005**
(21) Anmeldenummer: 96920675.4
(22) Anmeldetag: 05.07.1996
(51) Int. Cl.: A61M 5/315, A61M 5/24

(54) **INJEKTIONSGERÄT ZUM INJIZIEREN VON FLÜSSIGKEIT**
INJECTION DEVICE FOR INJECTION OF LIQUID
APPAREIL POUR L'INJECTION DE LIQUIDE

(43) Veröffentlichungstag der Anmeldung: 24.06.1998
(73) Patentinhaber: TecPharma Licensing AG, 3401 Burgdorf (CH)
(72) Erfinder: JOST, Stefan, CH-3065 Bolligen (CH); RENGGLI, Christoph, CH-3013 Bern (CH); BURKHARDT, Hans, CH-4500 Solothurn (CH)
(74) Vertreter: Schwabe - Sandmair - Marx
(86) Internationale Anmeldenummer: PCT/CH1996/000249
(87) Internationale Veröffentlichungsnummer: WO 1998/001172

(56) Entgegenhaltungen:
- EP-A- 0 554 995
- WO-A-91/10460
- US-A- 4 592 745

## Beschreibung

Die vorliegende Erfindung bezieht sich auf ein Injektionsgerät zum Injizieren von Flüssigkeiten gemäss dem Oberbegriff des Anspruchs 1.

Spritzenförmige Injektionsgeräte zum Injizieren von Flüssigkeiten sind seit längerem bekannt. Sie besitzen einen hülsenförmigen Hauptkörper, der etwa in der Mitte zusammenschraubbar ist und sich in zwei Hauptbereiche einteilen lässt:
einen proximalen (dem Patienten abgewandten) die Abgabemechanik beinhaltenden Bereich, mindestens bestehend aus einem stabförmigen Abriebsglied mit strukturierter Oberfläche (z.B. einer Schraubenspindel), einem zum Abtriebsglied passenden hohlzylindrischen Gegenelement mit einem strukturierten inneren Mantel (z.B. eine Schraubenmutter) und einem Bedienknopf; und
einen distalen (dem Patienten zugewandten) Bereich, in welchem sich die zu verabreichende Flüssigkeit und ein beweglicher Kolben befindet.

Am distalen Ende des Hauptkörpers wird eine Nadel mit Nadelhalter aufgesetzt, welche den Ausfluss der Flüssigkeit aus dem Gerät ermöglicht; bekannte solche Nadeln sind z.B. PENFINE ® Nadeln, wie in der WO 95/01812 beschrieben.

Verbindungsglied zwischen dem proximalen und distalen Bereich des Hauptkörpers ist das Abtriebsglied, welches den Kolben um die gewählte Dosis in distaler Richtung verschiebt, was zum Ausstoss der Flüssigkeit durch die Nadel führt.

Häufig befindet sich die zu verabreichende Flüssigkeit nicht direkt im Hauptkörper, sondern in einer Ampulle, wobei die Flüssigkeit zwischen einer Durchstechmembrane und einem gleitbeweglichen Kolben gelagert ist.

Je nach Injektionsgerät wird von der Abgabemechanik Unterschiedliches erwartet. Es gibt Geräte, welche lediglich eine einzige Ausschüttung, solche die mehrere gleichgrosse Ausschüttungen und solche, die mehrere frei wählbare Ausschüttungen zulassen.

Unabhängig davon, wie komplex die Abgabemechanik aufgebaut ist, ist es bei denjenigen Injektionsgeräten, welche ein Auswechseln der Ampulle zulassen, für den Patienten sehr aufwendig, wenn das Abtriebsglied in die Ausgangsposition zurückgedreht werden muss, um nach dem Auswechseln der Ampulle wieder betriebsbereit zu sein. Geräte, welche eine Rückdrehung des Abtriebgliedes mittels Bedienknopf verlangen, sind aus der WO 93/16740 bekannt.

Für den Patienten einfacher zu bedienen sind diejenigen Geräte, welche ein Rückschieben der Gewindestange erlauben, wie in den Schriften US-A-4 592 745 und EP-A-0 554 995 offenbart. Nachteilig an diesen bekannten Geräten ist, dass durch Lösung des distalen Bereichs vom proximalen Bereich des Hauptkörpers, die Gewindemutter gespreizt wird und dadurch die Gewindestange frei bewegbar ist, ohne dass dies vom Patienten eine bewusste Handlung verlangt, so dass ein Zusammenschrauben der beiden Bereiche des Hauptkörpers, nach dem Auswechseln der Ampulle, leicht zu einer vorzeitigen, ungewollten Ausschüttung der Flüssigkeit führen kann. Je nach Injektionsgerät kann auch eine Fehldosierung daraus resultieren, was bei gewissen Medikamenten sehr gefährlich für die Gesundheit eines Patienten ist.

Hier will die Erfindung Abhilfe schaffen. Der Erfindung liegt die Aufgabe zugrunde, ein Injektionsgerät anzugeben, bei welchem ein Rückschieben des Abtriebsgliedes vom Patienten eine bewusste Handlung verlangt, welche Abtriebsglied und Gegenelement voneinander löst, um die Gefahr einer Fehldosierung zu vermeiden.

Die Erfindung löst die gestellte Aufgabe mit einem Injektionsgerät, dessen Abtriebsglied, durch eine zwangsweise Öffnung des Gegenelementes, axial frei verschiebbar ist, wobei halbschalige Gewindemutterhälften das Gegenelement bilden.

Die durch die Erfindung erreichten Vorteile sind im Wesentlichen darin zu sehen, dass ein freies Zurückgleiten des Abtriebsgliedes nur dann möglich ist, wenn der Patient durch Betätigung einer Spreizhülse, also mit einer bewussten zusätzlichen Handlung, das Abtriebsglied freigibt.

Ein bevorzugtes Ausführungsbeispiel der Erfindung ist in den Figuren dargestellt.

Es zeigen:
- Fig. 1: ein erfindungsgemäßes Injektionsgerät mit gehaltenem Abtriebsglied;
- Fig. 2: ein erfindungsgemäßes Injektionsgerät mit freiem Abtriebsglied;
- Fig. 3: ein erfindungsgemäßes Injektionsgerät mit entfernter Betätigungseinrichtung;
- Fig. 4: einen Querschnitt durch die Linie A-A;
- Fig. 5: einen Querschnitt durch die Linie B-B; und
- Fig. 6: einen Querschnitt durch die Linie C-C.

In der nachfolgenden Beschreibung werden die Begriffe proximal und distal in der in der Medizin üblichen Weise gebraucht, also proximal = dem Patienten abgewandt und distal = dem Patienten zugewandt.

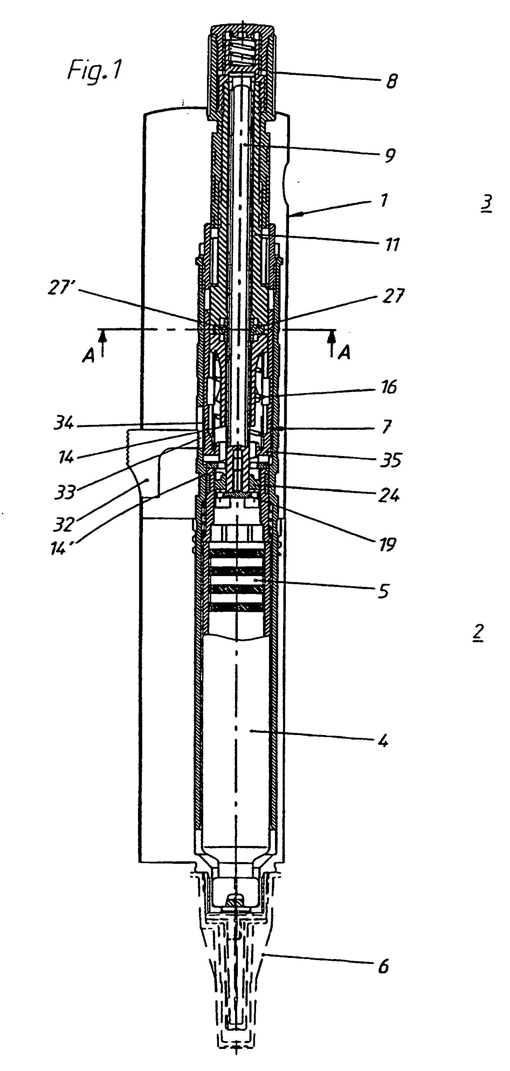

Wie in Fig. 1 dargestellt, besteht das erfindungsgemässe Injektionsgerät aus einem hülsenförmigen Hauptkörper 1, der sich in einen hinteren (proximalen) Bereich 3, welcher die röhrenförmige Betätigungseinrichtung oder Ausschüttmechanik 7 beinhaltet und einen vorderen (distalen) Bereich 2, der eine austauschbare Ampulle 4 mit einem Kolben 5 beinhaltet, einteilen lässt. Auf dem distalen Ende des Hauptkörpers 1 kann eine Nadel 6 aufgeschraubt werden, dessen proximales Ende mit der zu verabreichenden Flüssigkeit in Verbindung steht. Die Betätigungseinrichtung 7 weist einen Bedienknopf 8, ein Abtriebsglied 9, mit einem Flansch 19, ein Führungselement 24 und ein Antriebselement 11 auf.

Das röhrenförmige Antriebselement 11 ist mit dem Bedienknopf 8 fest gegen Verdrehung verbunden. Mit seinem distalen Ende umhüllt das Antriebsglied 11 zwei Gewindeflansche 27,27', welche in das Gewinde des Abtriebglieds 9 eingreifen.

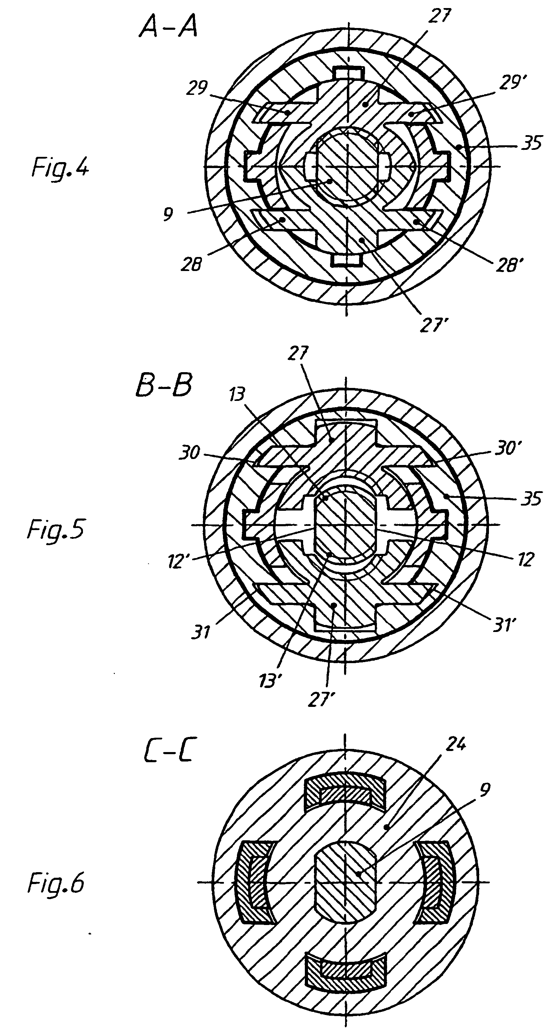

Aus Figur 5 ist ersichtlich, dass das Abtriebsglied 9 zwei plane Flächen 12,12' besitzt, im übrigen aber einen kreisförmigen Querschnitt aufweist und auf den kreisförmigen Flächen 13,13' ein Gewinde trägt.

Das Führungselement 24 ist sowohl gegen Rotationsbewegungen, wie auch gegen axiale Bewegung fest mit dem proximalen Bereich 3 des Hauptkörpers 1 verbunden und liegt unterhalb des Antriebselements 11. Die Öffnung im Führungselement 24, durch welche das Abtriebsglied 9 hindurchgeht (Fig. 6), besitzt denselben Querschnitt - um gewisse Toleranzen vergrössert - wie der Querschnitt des Abtriebglieds 9. Da das Führungselement 24, im Gegensatz zum Antriebselement 11, kein Gewinde aufweist, kann das Abtriebsglied 9 durch diese Öffnung des Führungselementes in axialer Richtung hin- und hergeschoben werden. Eine Rotationsbewegung des Abtriebglieds 9 ist somit nicht möglich, weil dies das Führungselement 24 nicht zulässt.

Am Bedienknopf 8 können sowohl axiale, wie auch rotierende Bewegungen vorgenommen werden. Wird er durch Druck in distaler Richtung betätigt, so verschiebt er gleichzeitig das Antriebselement 11 bis dessen vordere Stirnfläche 14 auf die hintere Stirnfläche 14' des Führungselementes auftrifft.

Das Abtriebsglied 9 ist mit dem Antriebselement 11 durch die Gegenelemente (Gewindeflansche) 27,27' verbunden, so dass axiale Bewegungen des Bedienknopfes 8 übertragen werden können. Eine detaillierte Beschreibung der Gewindeflansche 27,27' erfolgt weiter unten.

Die axiale Bewegung wird entgegen der Kraft einer Feder 16 ausgeführt, welche die Betätigungseinrichtung 7 wieder in die Ruhestellung (Fig. 1) zurückbringt.

Wird der Bedienknopf 8 zur Einstellung einer Injektionsdosis gedreht, so dreht sich das Antriebselement 11 mit ihm. Diese Drehbewegung kann aber nicht auf das Abtriebselement 9 übertragen werden, da diese im Führungselement 24 drehfest gelagert ist. Durch die sich drehenden Gegenelemente 27,27' im Antriebselement 11 wird, über die Gewindeteile an den Kreisflächen 13,13' der Gewindestange 9 diese drehfest nach vorne (oder bei Umkehr der Drehsicherung am Bedienknopf 8 nach hinten) getrieben, und so der Flansch 19 in diejenige Stellung gebracht, welche die nächste abzugebende Injektionsdosis erfordert, d.h. der Abstand des Flansches 19 vom Kolben 5 wird entsprechend verringert.

Durch Druck auf den Bedienknopf 8 wird die Betätigungseinrichtung 7 aus der Ruhestellung in die Endlage vorgeschoben. Der Flansch 19 stösst dabei am Kolben 5 an und nimmt ihn auf dem eingestellten Kolbenweg mit, wodurch das im voraus eingestellte Volumen der Injektionsflüssigkeit durch die Injektionsnadel 6 ausgestossen wird. Der Weg des Flansches 19 von der Ruhelage in die Endlage der Betätigungseinrichtung 7 bleibt immer derselbe und entspricht einer konstanten Distanz, um die der Flansch 19 vor Einstellung der Injektionsdosis vom Kolben 5 getrennt ist. Dieser Vorgang ist im Detail in der WO 93/16740 beschrieben.

Ist der Flüssigkeitsbehälter 4 leer und befindet sich demnach das Abtriebsglied 9 in distaler, unterster Position, muss die Gewindestange wieder in ihre proximale, oberste Position gebracht werden. Das erfindungsgemässe Injektionsgerät 1 erlaubt eine Rückschiebung des Abtriebglieds 9 durch Betätigung eines Entriegelungsschiebers 32.

Aus den Fig. 4 und 5 ist ersichtlich, dass die beiden Gegenelemente 27,27' des Antriebselements 11 als zwei halbschalige Gewindemutterhälften 27,27' ausgestaltet sind, welche je zwei Nocken 28,28', 29,29' aufweisen.

Der am hinteren Bereich 3 befestigte Entriegelungsschieber 32 ist mit einer Spreizhülse 35 in Inneren des hinteren Bereichs 3 derart verbunden, dass ein Verschieben des Entriegelungsschiebers 32 in proximaler Richtung, zu einem Verschieben der Spreizhülse 35 proximaler Richtung führt.

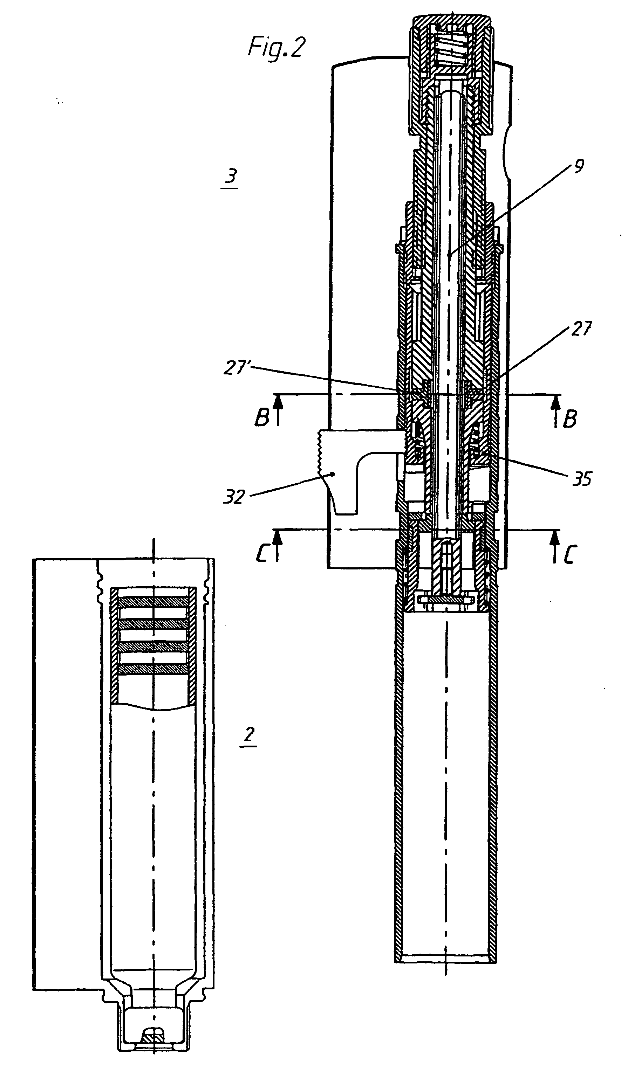

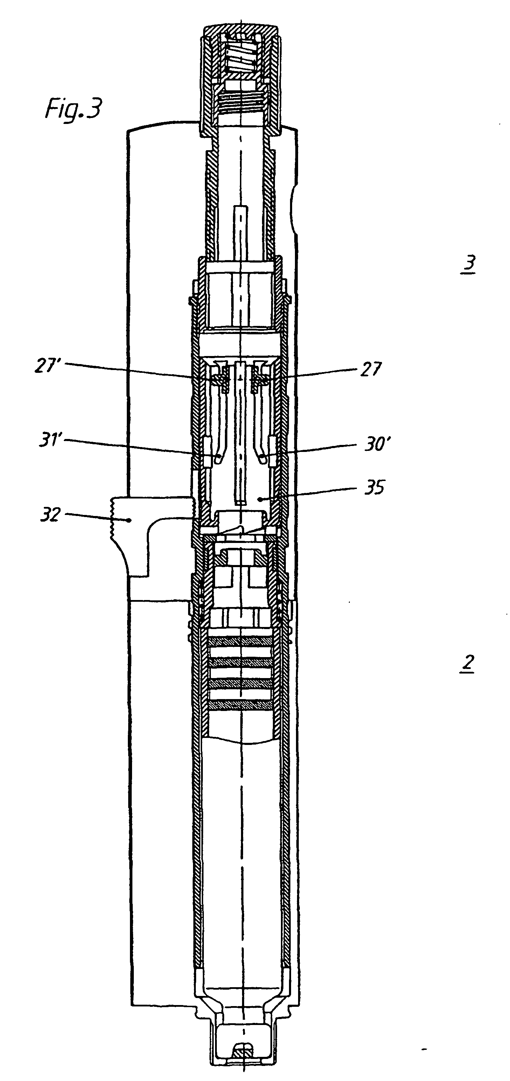

Die Spreizhülse 35 umhüllt die Antriebselements 11 und besitzt vier senkrecht verlaufende Bahnen 30,30', 31,31' (Fig. 3), welche gegen das distale Ende der Spreizhülse 35 unter einem Winkel gegen aussen verlaufen. Die Bahnen 30,30', 31,31' dienen zur Aufnahme der Nocken 28,28', 29,29' der Gegenelemente 27,27'. Befindet sich die Spreizhülse 35 in distaler Position (Fig. 1), umschliessen die Gegenelemente 27,27' das Abtriebsglied 9. Wird mit dem Entriegelungsschieber 32 die Spreizhülse 35 in ihr proximale Position (Fig. 2) geschoben, so öffnen sich die Gegenelemente 27,27', sobald ihre Nocken 28,28', 29,29' über die Winkel der Bahnen 30,30', 31,31' laufen und das Abtriebsglied 9 ist axial frei verschiebbar.

Eine Rasterung 33 des Entriegelungsschiebers 32, welcher am Hauptkörper angebracht ist, passt in eine Gegenrasterung 34, am distalen Teil der Spreizhülse 35.

Grundsätzlich wird die Spreizhülse 35 durch die Feder 16 in ihrer distalen Position festgehalten. Soll nun das Gegenelement 27,27' gelöst werden, so wird durch den Benutzer der Entriegelungsschieber 32 aktiv, durch gleichzeitiges Hineindrücken, in seine proximale Position geschoben. Bei diesem Vorgang greift die Rasterung 33 des Entriegelungsschiebers 32 in die Gegenrasterung 34 der Spreizhülse 35 ein und schiebt diese nach hinten. Durch diese Bewegung laufen die Nocken 28,28', 29,29' zwangsweise über die entsprechenden, nach aussen laufenden Bahnen 30,30', 31,31' (Fig. 3 - 5) der Spreizhülse 35. Diese zwangsweise Bewegung hat zur Folge, dass sich die Gegenelemente 27,27' öffnen (Fig. 5) und das Abtriebsglied 9 freigegeben wird. Wird nun das Injektionsgerät gleichzeitig mit dem Bedienknopf 8 nach unten gehalten, fällt das Abtriebsglied 9 durch die Schwerkraft selbstständig in ihre proximale Position.
Durch Loslassen des Entriegelungsschiebers 32 gleitet die Spreizhülse 35 wieder nach vorne. Dabei gleiten die Nocken 28,28', 29,29' in den Bahnen 30,30', 31,31' zurück in ihre Halteposition, in welcher das Gegenelement 27,27' geschlossen ist. Der Entriegelungsschieber 32 wird durch - nicht gezeichnete - Mittel in die distale Position gebracht.

Aus Sicherheitsgründen können die Gegenelemente 27,27' nur von dem Abtriebsglied 9 gelöst werden, wenn die Betätigungseinrichtung 7 in ihrer distalen Stellung ist (Fig. 2). Aus diesem Grund sind die Bahnen 30,30', 31,31' so bemessen, dass eine Betätigung des Entriegelungsschiebers 32 in der Arbeitsstellung der Betätigungseinrichtung 7 (Fig. 1 und 3) lediglich zu einem Gleiten der Nocken 28,28', 29,29' in den senkrechten Bahnen 30,30', 31,31' führt, ohne deren abgewinkelten Bereich, am distalen Ende der Spreizhülse 35, zu erreichen. Damit die Nocken 28,28', 29,29' den abgewinkelten Bereich der Bahnen 30,30', 31,31' erreichen und sich von dem Abtriebsglied 9 lösen können, muss zusätzlich die Betätigungseinrichtung 7 in ihrer distalen, untersten Position (Fig. 2) sein.

Denkbar sind auch einfachere Antriebsmechanismen, bei denen das Abtriebsglied 9 nicht als Gewindestange, sondern mit einer sägezahnartigen Struktur, ausgestaltet ist.

### Verzeichnis der Merkmale zu W0 98/01172

- 1: Hauptkörper
- 2: vorderer distaler Bereich
- 3: hinterer proximaler Bereich
- 4: Ampulle
- 5: Kolben
- 6: Nadel
- 7: Betätigungseinrichtung (Abgabemechanik)
- 8: Bedienknopf, Dosierknopf
- 9: Abtriebsglied (Gewindestange)
- 11: Antriebselement (Gewindemutter)
- 12, 12: plane Flächen des Abtriebsglieds
- 13, 13: kreisförmige Flächen des Abtriebsglieds
- 16: Feder der Spreizhülse
- 19: Flansch an dem Abtriebsglied
- 24: Führungselement
- 27, 27': Gegenelemente des Antriebselements, Gewindeflansche oder Teilschale
- 28, 28; 29,29': Nocken an den Gegenelementen
- 30,30'; 31,31': Bahnen der Spreizhülse
- 32: Entriegelungsschieber
- 33: Rasterung des Entriegelungsschiebers
- 34: Gegenrastung an der Spreizhülse
- 35: Spreizhülse

## Patentansprüche

1. Injektionsgerät (1) zum Injizieren von Flüssigkeiten aus einem Flüssigkeitsbehälter (4), der mit einem Kolben (5) ausgerüstet ist,
a) mit einer Betätigungseinrichtung (7), die ein stabförmiges, axial geführtes Abtriebsglied (9) umfasst, das mit einem Gewinde versehen ist,
b) mit einem Bedienknopf (8), an dem axiale Bewegungen ausführbar sind,
c) und mit zum Abtriebsglied (9) passenden, dazu koaxial angeordneten, halbschaligen Gewindemutterhälften (27, 27'),
**dadurch gekennzeichnet, dass**
d) die Gewindemutterhälften (27, 27') Nocken (28,28',29,29') aufweisen,
e) und dass eine axial verschiebbare Spreizhülse (35) vorgesehen ist, die Bahnen (30, 30', 31, 31') zur Führung der Nocken (28, 28', 29, 29') aufweist,
f) wobei die Bahnen (30, 30', 31, 31') in einem proximalen Bereich axial und in einem distalen Bereich unter einem Winkel zur Axialen verlaufen, so dass die Gewindemutterhälften (27, 27') durch axiales Verschieben der Spreizhülse (35) zwangsweise derart geöffnet werden, dass das Abtriebselement (9) darin axial frei verschiebbar ist.

2. Injektionsgerät nach Anspruch 1, **dadurch gekennzeichnet, dass** die Gewindemutterhälften (27, 27') je zwei der Nocken (28, 28', 29, 29') aufweisen.

3. Injektionsgerät nach einem der Ansprüche 1 und 2, **dadurch gekennzeichnet, dass** das Abtriebsglied (9) auf zwei gegenüberliegenden Seiten plane Flächen (12, 12'), im übrigen aber Kreisflächen (13, 13') mit Gewinden aufweist.

4. Injektionsgerät nach einem der Ansprüche 1 bis 3, **dadurch gekennzeichnet, dass** das Öffnen der Gewindemutterhälften (27, 27') nur möglich ist, wenn sich die Gewindemutterhälften (27, 27') in distaler Position befinden.

5. Injektionsgerät nach einem der Ansprüche 1 bis 4, **dadurch gekennzeichnet, dass** die Spreizhülse (35) mit einem Entriegelungsschieber (32) verbindbar ist.

6. Injektionsgerät nach einem der Ansprüche 1 bis 5, **dadurch gekennzeichnet, dass** die Bahnen (30, 30', 31, 31') in dem distalen Bereich unter einem Winkel nach außen verlaufen, so dass durch axiales Verschieben der Spreizhülse (35) in Richtung zu dem proximalen Ende die Gewindemutterhälften (27, 27') zwangsweise geöffnet werden.

## Claims

1. An injection device (1) for injecting fluids from a fluid container (4) equipped with a piston (5), comprising:
a) an actuating device (7) having a rod-shaped, axially guided driven member (9) provided with a thread;
b) a control button (8) on which axial movements may be performed;
c) and hemispherical threaded half-nuts (27, 27') corresponding to the driven member (9) and arranged co-axially with respect to the driven member (9),
**characterised in that**
d) the threaded half-nuts (27, 27') comprise cams (28, 28', 29, 29'),
e) and **in that** a spreader bushing (35) is provided which can be axially shifted and comprises tracks (30, 30', 31, 31') for guiding the cams (28, 28', 29, 29'),
f) wherein the tracks (30, 30', 31, 31') run axially in a proximal area and at an angle to the axial in a distal area, such that the threaded half-nuts (27, 27') are forced open by axially shifting the spreader bushing (35), such that the driven member (9) can be freely shifted axially therein.

2. The injection device as set forth in claim 1, **characterised in that** the threaded half-nuts (27, 27') each comprise two of the cams (28, 28', 29, 29').

3. The injection device as set forth in any one of claims 1 and 2, **characterised in that** the driven member (9) comprises level surfaces (12, 12') on two facing sides, but otherwise threaded circular surfaces (13, 13').

4. The injection device as set forth in any one of claims 1 to 3, **characterised in that** the threaded half-nuts (27, 27') can only be opened when the threaded half-nuts (27, 27') are in the distal position.

5. The injection device as set forth in any one of claims 1 to 4, **characterised in that** the spreader bushing (35) can be connected to an unlocking slide (32).

6. The injection device as set forth in any one of claims 1 to 5, **characterised in that** the tracks (30, 30', 31, 31') run outwards at an angle in the distal area, such that the threaded half-nuts (27, 27') are forced open by axially shifting the spreader bushing (35) towards the proximal end.

## Revendications

1. Appareil d'injection pour l'injection de liquides à partir d'un récipient à liquide (4), qui est équipé d'un piston (5), comportant
a) un dispositif d'actionnement (7), qui comprend un organe entraîné en forme de tige (9) guidé axialement, qui est pourvu d'un filetage,
b) un bouton d'actionnement (8), auquel des déplacements axiaux peuvent être appliqués,
c) et des moitiés d'écrou (27, 27') en forme de demi-coques qui sont adaptées à l'organe entraîné (9) et sont disposées coaxialement à ce dernier;
**caractérisé en ce que**
d) les moitiés (27, 27') de l'écrou comportent des cames (28, 28', 29, 29'),
e) et qu'il est prévu une douille d'écartement (31) déplaçable axialement, qui comporte des pistes (30, 30', 31, 31') pour le guidage des cames (28, 28', 29, 29'),
f) les pistes (30, 30', 31, 31') s'étendant axialement dans une zone proximale, et en faisant un angle par rapport à l'axe dans une zone distale, de sorte que les moitiés (27, 27') de l'écrou peuvent être ouvertes de façon forcée par déplacement axial de la douille d'écartement (35) de telle sorte que l'élément entraîné (9) y est librement déplaçable axialement.

2. Appareil d'injection selon la revendication 1, **caractérisé en ce que** les moitiés (27, 27') de l'écrou comportent chacune deux des cames (28, 28', 29, 29').

3. Appareil d'injection selon l'une des revendications 1 et 2, **caractérisé en ce que** l'organe entraîné (9) comporte, sur deux côtés opposés, des surfaces planes (12, 12'), mais comporte ailleurs des surfaces circulaires (13, 13') pourvues de filetages.

4. Appareil d'injection selon l'une des revendications 1 à 3, **caractérisé en ce que** l'ouverture des moitiés (27, 27') de l'écrou est possible uniquement lorsque les moitiés (27, 27') de l'écrou sont situées dans une position distale.

5. Appareil d'injection selon l'une des revendications 1 à 4, **caractérisé en ce que** la douille d'écartement (35) peut être reliée à un poussoir de déverrouillage (32).

6. Appareil d'injection selon l'une des revendications 1 à 5, **caractérisé en ce que** les pistes (30, 30', 31, 31') s'étendent, dans la zone distale, sous un angle en direction de l'extérieur de sorte que sous l'effet d'un déplacement axial de la douille d'écartement (35) en direction de l'extrémité proximale, les moitiés (27, 27') de l'écrou sont ouvertes de façon forcée.
